# EUROPÄISCHE PATENTSCHRIFT

(11) **EP 3 346 936 B1**
(45) Veröffentlichungstag und Bekanntmachung des Hinweises auf die Patenterteilung: **10.04.2019**
(21) Anmeldenummer: 17777195.3
(22) Anmeldetag: 15.09.2017
(51) Int. Cl.: A61B 17/70

(54) **STABEINBRINGINSTRUMENT MIT VERSTELLBARER STABANGULIERUNG**
ROD INTRODUCING INSTRUMENT WITH ADJUSTABLE ROD ANGLING
INSTRUMENT D'INSERTION DE LA BARRE À ANGLE DE LA BARRE RÉGLABLE

(30) Priorität: 23.09.2016 DE 102016011521
(43) Veröffentlichungstag der Anmeldung: 18.07.2018
(73) Patentinhaber: Silony Medical International AG, 8500 Frauenfeld (CH)
(72) Erfinder: RIESINGER, Ralf, 78532 Tuttlingen-Nendingen (DE)
(74) Vertreter: Stolmár & Partner Patentanwälte PartG mbB
(86) Internationale Anmeldenummer: PCT/EP2017/073272
(87) Internationale Veröffentlichungsnummer: WO 2018/054774

(56) Entgegenhaltungen:
- WO-A1-2008/130548
- WO-A2-2011/109507
- US-A1- 2014 277 166
- US-A1- 2016 143 674

## Beschreibung

### Gebiet der Erfindung

Die Erfindung betrifft ein Stabeinbringinstrument mit verstellbarer Stabangulierung zum Einbringen eines Verbindungsstabs für Pedikelschrauben.

### Stand der Technik

Im Stand der Technik werden üblicherweise Stabeinbringinstrumente verwendet, die den Verbindungsstab am Ende in einer vorgegebenen Position halten. So wird der Verbindungsstab dann durch zwei benachbart liegende Pedikelschrauben hindurch geschoben. Im Stand der Technik werden die Verbindungsstäbe beispielsweise über Schraubmechanismen fixiert. So ist in der DE 10 2013 107 308 A1 ein Stabeinsetzinstrument zum Einsetzen eines Verbindungsstabes in die Tulpen benachbarter Pedikelschrauben beschrieben, der ein Griffteil, ein sich daran anschließenden Rohrabschnitt und einem vom Rohrabschnitt getragenen Greifkopf offenbart, in dem der Verbindungsstab mit seinem proximalen Ende drehfest und winkelstabil aufnehmbar ist, wobei die Verbindung zwischen Verbindungsstab und Greifkopf mittels eines manuell antreibbaren Schiebekörpers lösbar ist. Der Greifkopf am Rohrabschnitt ist ausschließlich über ein sperrbares Gelenk angebracht, mit welchem dem Rohrabschnitt beim Fixiervorgang des Verbindungsstabs an der Pedikelschraube eine fesselungsfreie Schwenkbewegung auf den Verbindungsstab ermöglicht. Der Verbindungsstab wird hier über eine Schraube befestigt. Eine solche Schraube bedeutet jedoch beim Befestigen und vor allem beim Lösen des Verbindungsstabs nach der Operation einen gewissen Aufwand und zwingt den Anwender seine Händen nahe an den eingesetzten Verbindungsstab zum Ausschrauben der Befestigung zu bringen. Dadurch kann auch ein erhöhtes Risiko für eine Verletzung am Patienten entstehen.

In der WO 2008/130548 A1 ist ein anderes Instrument gezeigt, das den Stab in einer bestimmten Winkelposition halten kann. Dazu wird der Stab erst von L-förmigen Greifern erfasst und dann über eine Schubstange in die gewünschte Winkelposition geschoben. Der Betätigungshebel für die Schubstange kann jedoch aus Versehen während der Operation weiter festgezogen werden, was die Winkelstellung des Stabs verändert. Der US2016/0143674, offenbart ein Stabeinbringvorrichtung zum Einbringen eines Verbindungsstabs in eine Pedikelschraube, umfassend einen Aufnahmekörper, der an seinem distalen Ende eine Aufnahmeaussparung einen in dem Aufnahmekörper angeordneten Klemmkörper zum Festklemmen des Verbindungsstabs in der Aufnahmeaussparung.

### Darstellung der Erfindung

Die Aufgabe der vorliegenden Erfindung ist es, ein Stabeinsetzinstrument bereitzustellen, das zum einen eine einfache und flexible Verstellbarkeit des Verbindungsstabs am Stabeinsetzinstrument bereitstellt, gleichzeitig aber eine stabile, sichere aber dennoch leicht zu öffnende Fixierung des Verbindungsstabs ermöglicht.

Eine erfindungsgemäße Stabeinbringvorrichtung zum Einbringen eines Verbindungsstabs in eine Pedikelschraube, umfasst einen insbesondere länglichen Aufnahmekörper, der an seinem distalen Ende eine Aufnahmeaussparung mit gegenüberliegenden Seitennuten aufweist, einen in dem Aufnahmekörper verschiebbar angeordneten Klemmkörper zum Festklemmen des Querstabs in der Aufnahmeaussparung der Stabeinbringvorrichtung, wobei die insbesondere hakenförmigen oder kurvenförmigen Seitennuten der Aufnahmeaussparung in einem Eingangsbereich in einer ersten Richtung in dem Aufnahmekörper ausgebildet sind und in einem Festsitzbereich nach dem Eingangsbereich in einer zweiten Richtung unterschiedlich zur ersten Richtung ausgebildet ist, so dass ein eingesetzter Verbindungsstab in der ersten Richtung blockiert ist und/oder bei der ferner ein Sperrelement in dem Aufnahmekörper vorgesehen ist, das insbesondere entlang der Eingangsrichtung der Aufnahmeaussparung zwischen einer Freigabeposition und einer Sperrposition bewegbar und insbesondere translatorisch verschiebbar gelagert ist. Sowohl durch die Seitennut mit Richtungsänderung als auch durch das Sperrelement und insbesondere durch deren Kombination kann sichergestellt werden, dass der Verbindungsstab nicht mehr versehentlich aus der Einsetzposition in der Stabeinbringvorrichtung herausfallen kann. Insbesondere ist die Seitennut zumindest teilweise als Durchgangsbohrung ausgebildet. Dadurch ist die Herstellung der Seitennut insbesondere falls ein zweiter Bereich in einer zweiten Richtung vorgesehen ist, sehr einfach.

Vorzugsweise ist bei der Stabeinbringvorrichtung an dem distalen Ende des Sperrelements zumindest ein Sperrvorsprung vorgesehen, der in der Sperrposition in die Aufnahmenut hereinragt. Ein solcher über die Bolzen des Verbindungsstabs in die Seitennut hereinragender Vorsprung eignet sich hervorragend um den Verbindungsstab in Position zu halten, ohne ihn gleichzeitig rotatorisch in einer Winkelstellung festzulegen. Die Winkelstellung betrifft die relative Lage des Verbindungsstabs zur Stabeinbringvorrichtung.

Bei der Stabeinbringvorrichtung ist das Sperrelement in dem Aufnahmekörper insbesondere federnd gelagert und in die Sperrposition vorgespannt. Dadurch kann abgesichert werden, dass das Sperrelement auch zuverlässig in seiner Sperrposition sitzt auch ohne dass der Anwender dies überprüfen muss.

Das Sperrelement weist am proximalen Ende einen Schiebehebel zum Verschieben auf. Mit diesem Hebel kann das Sperrelement einfach und einhändig betätigt werden, ohne dass der Anwender direkt am distalen Ende der Stabeinbringvorrichtung das Risiko eingehen muss, an der Operationsstelle versehentlich etwas zu berühren. Gerade beim Lösen des Verbindungsstabs aus der Stabeinbringvorrichtung ist diese Risikominimierung äußerst hilfreich.

Vorzugsweise weist der Klemmkörper der Stabeinbringvorrichtung an seinem distalen Ende eine sich verjüngende Geometrie, insbesondere eine Spitze oder einen Zahn, auf oder ist zahnförmig ausgebildet. Die zahnförmige Ausbildung ist vorzugsweise an einer nach innen gewölbten konkaven Fläche am distalen Ende des Klemmkörpers ausgebildet. Die Winkelstellung des Verbindungsstabs kann so sehr einfach fixiert werden, da das proximale Ende des Verbindungsstabs an einer kleinen Stelle mit einem hohen Druck belastet wird und der Verbindungsstab sich dadurch nicht mehr drehen kann. In einer anderen Ausgestaltung ist der Klemmkörper an seinem distalen Ende Halbkreisförmig ausgebildet und weist insbesondere einen oder mehrere Zähne wie bei einem Zahnrad auf. Wenn das proximale Ende des Verbindungsstabs dann komplementär ausgebildet ist, kann die Winkelstellung des Verbindungsstabs sehr einfach und vor allem zuverlässig fixiert werden.

Vorzugsweise ist der Aufnahmekörper, der Klemmkörper und insbesondere auch das Sperrelement als sich längserstreckende Körper, insbesondere als Zylinderkörper mit einer beliebigen Außengeometrie (kreisförmig, elliptisch, rechteckig, quadratisch, hexagonal, usw.) ausgebildet. Durch diese Ausgestaltung ist es einfach möglich, die verschiedenen Körper ineinander bzw. umeinander anzuordnen.

Der Klemmkörper ist vorzugsweise im Sperrelement und das Sperrelement in dem Aufnahmekörper angeordnet. Dadurch ist eine platzsparende Ausgestaltung gewährleistet,

Stabeinbringvorrichtung nach einem der vorhergehenden Ansprüche, bei der am Aufnahmekörper ein Haltegriff in Pistolenform ausgebildet ist. Mit dieser ergonomischen Form des Haltegriffs ist insbesondere bei winkeliger Anordnung des Verbindungsstabs ein Einsetzten des Stabs leichter möglich. Außerdem ist dadurch auch die einhändige Betätigung der verschiedenen Funktionen am proximalen Ende einfach durchzuführen.

Die Stabeinbringvorrichtung weist bevorzugt am Ende des Aufnahmekörpers am proximalen Ende eine verschließbare Montageöffnung auf. So ist die Stabeinbringvorrichtung leichter zu Montieren und Demontieren und damit auch für die Wiederverwendung leichter zu Reinigen. Insbesondere ist der Klemmkörper mittels eines Betätigungshebels gegen einen Klemmbereich des Verbindungsstabs pressbar. Dadurch kann der Klemmkörper mit einer verstärkten Kraft an den Verbindungsstab gedrückt werden, was die Festigkeit und die Sicherheit mit der der Verbindungsstab festgehalten wird erhöht.

Der Klemmkörper und das Sperrelement sind vorzugsweise über einen Verschluss im Aufnahmekörper gesichert Das erhöht die Sicherheit gegen ein zufälliges oder versehentliches Demontieren der Einzelteile der Stabeinbringvorrichtung. Insbesondere sind beide auch über das Öffnen des Verschlusses für Reinigungszwecke demontierbar. Dadurch wird eine einfache Handhabe für die Reinigung bereitgestellt.

### Kurze Beschreibung der Figuren

Figur 1 zeigt eine isometrische Ansicht einer erfindungsgemäßen Stabeinbringvorrichtung;
Figur 2 zeigt eine Vergrößerung des distalen Endes der erfindungsgemäßen Stabeinbringvorrichtung aus Figur 1;
Figur 3 zeigt eine Explosionsansicht der erfindungsgemäßen Stabeinbringvorrichtung aus Figur 1;
Figuren 4 bis 6 zeigen Vergrößerungen der distalen Enden der Einzelteile der erfindungsgemäßen Stabeinbringvorrichtung aus Figur 3;
Figur 7 zeigt einen Längsschnitt der erfindungsgemäßen Stabeinbringvorrichtung aus Figur 1; und
Figur 8 zeigt eine Draufsicht der erfindungsgemäßen Stabeinbringvorrichtung aus Figur 1.

### Beschreibung der bevorzugten Ausführungsformen

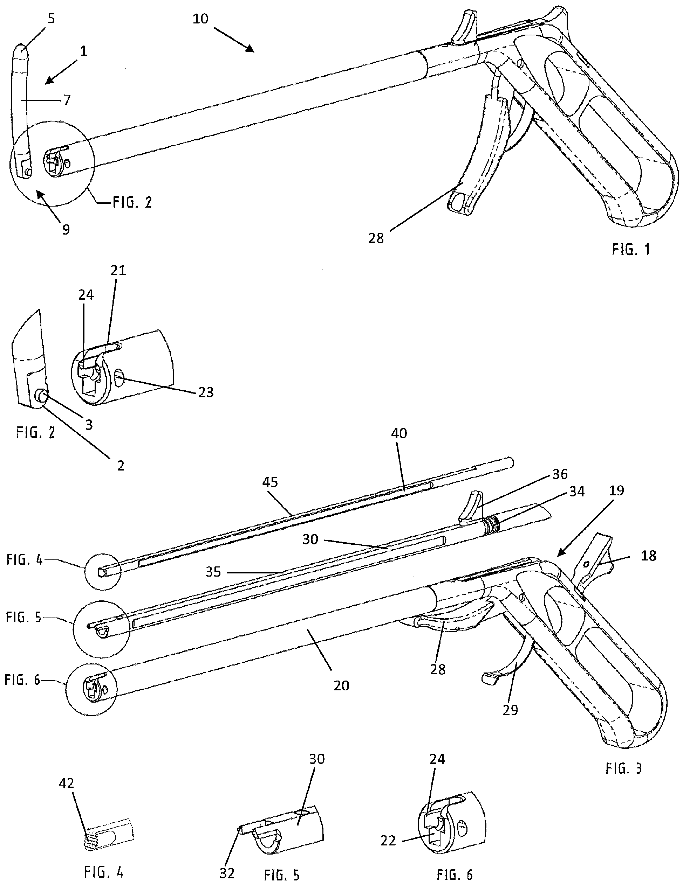

Im Folgenden werden die Begriffe axial, radial und umfänglich sowie distal und proximal verwendet. Axial bedeutet dabei eine Richtung entlang der Längsachse des in Figur 1 gezeigten Instruments, radial eine Richtung senkrecht zu dieser Längsachse und umfänglich eine Richtung um die Längsachse herum. Ferner werden die Bezeichnungen oben, unten, links und rechts ebenfalls in Bezug auf das in Figur 1 dargestellte Instrument verwendet. Distal bezeichnet eine Seite, die vom Anwender weg zeigt und proximal die zum Anwender gerichtete Seite.

In Figur 1 ist die bevorzugte Ausführungsform der Stabeinbringvorrichtung 10 dargestellt. Im Wesentlichen besteht die Stabeinbringvorrichtung aus einem länglichen Hauptkörper, der als Aufnahmekörper 20 ausgebildet ist und an einem distalen Ende eine Aufnahmeeinrichtung 22 für einen Verbindungsstab 1 aufweist und am anderen proximalen Ende einen Handgriff 12 zum Betätigen und Führen des Instruments.

Der Verbindungsstab 1 ist in den Figuren 1, 7 und 8 zu erkennen. Er ist dort als leicht gebogener Stab ausgebildet, der eine konische Spitze 5, einen gebogenen Abschnitt 7 und einen Halteabschnitt 9 aufweist. Der Halteabschnitt 9 ist an zwei gegenüberliegenden Seiten abgeflacht ausgebildet, wobei an den flachen Seiten Bolzen 3 hervorstehen. Der Halteabschnitt 9 wird dann von der Stabeinbringvorrichtung gehalten.

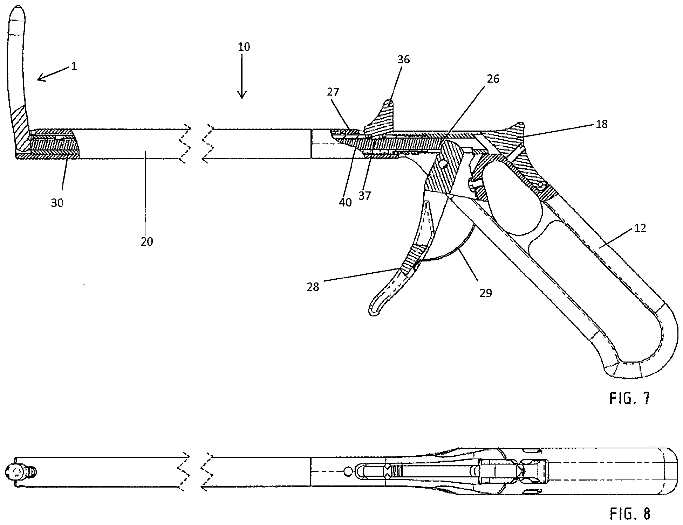

Die Stabeinbringvorrichtung 10 umfasst in der vorliegenden Ausführungsform den Aufnahmekörper 20, einen in dem Aufnahmekörper 20 angeordnetes Sperrelement 30 und einen in dem Sperrelement 30 angeordneten einen Klemmkörper 40 zum Festklemmen des Verbindungsstabs 1 in der Stabeinbringvorrichtung 10. Der Aufnahmekörper 20, das Sperrelement 30 und der Klemmkörper 40 sind in der vorliegenden Ausführungsform länglich ausgeführt. Dadurch kann die Stabeinbringvorrichtung 10 lang genug ausgeführt werden, um eine sichere Operation durchzuführen. Der Handgriff kann beliebig ausgeführt sein, im vorliegenden Fall ist er jedoch als Pistolengriff ausgebildet, steht also in einem Winkel zum Rest des Aufnahmekörpers 20. In dem Aufnahmekörper 20 ist der Klemmkörper 40 verschiebbar vorgesehen und wird am Handgriff mit einem Betätigungshebel 28 betätigt Der Betätigungshebel ist vorzugsweise in einer Betätigungsstellung festsetzbar. Eine stufenlose Feststelleinrichtung kann beispielsweise mit einer Gewindespindel und einer entsprechenden Feststellmutter ausgebildet sein, eine schrittweise Feststelleinrichtung kann als Zahnstange oder Raststange ausgebildet sein. Die Feststelleinrichtung sollte jedoch zumindest vom Betätigungshebel und/oder vom Handgriff lösbar sein, so dass der Betätigungshebel für die später beschriebene Montage frei nach oben geklappt werden kann, wie es in Figur 3 gezeigt ist. In Figur 7 ist der Betätigungshebel 28 gezeigt. Wenn der Handgriff in der Hand des Anwenders ist, können Zeigefinger und Mittelfinger den Betätigungshebel an der Kontaktstelle 26 gegen den hinteren Teil des Klemmkörpers drücken und den Klemmkörper mit durch den Hebelarm verstärkter Kraft gegen den Verbindungsstab 1 pressen. Dadurch klemmt der Klemmkörper 40 am distalen Ende dann einen in die Aufnahmeaussparung 22 eingebrachten Verbindungsstab 1 fest. Der Betätigungshebel 28 ist mit einer Feder 29 vorgespannt, die den Betätigungshebel 28 wieder in die entspannte Position drückt.

Die Aufnahmeaussparung 22 ist am vorderen, distalen Ende der Stabeinbringvorrichtung 10 ausgebildet. Die Aufnahmeaussparung 22 kann aber auch umfänglich am distalen Ende vorgesehen sein, beispielsweise auf der Oberseite. Das Sperrelement 30 und die seitliche nachfolgend beschriebenen Führungsnuten 24 müssen dann entsprechend angepasst werden. Die Aufnahmeaussparung 22 weist seitlich jeweils eine Führungsnut 24 für die Führungsbolzen des Verbindungsstab auf. Die Führungsnut 24 verläuft beim Eingang in einer ersten Richtung und macht dann eine Biegung. In der gezeigten Ausführungsform verläuft die Führungsnut 24 in dem ersten Bereich entlang der Längsachse des Aufnahmeelements und danach nach unten, so dass die Führungsnuten 24 hakenförmig oder kurvenförmig verlaufen, bspw. wie bei einem Bajonettverschluss. Durch diese Richtungsänderung der Führungsnuten 24 wird es dem Verbindungsstab erschwert, versehentlich aus der Nut zu fallen wenn er in seiner Endposition sitzt und von dem Klemmkörper 40 und/oder dem Sperrelement 30 festgesetzt ist. Damit der Verbindungsstab 1 in der Stabeinbringvorrichtung 10 so frei wie möglich bewegbar ist um verschiedene Winkelstellungen erreichen zu können, ist insbesondere auf der Oberseite an der Aufnahmeaussparung 22 ein Schlitz 21 vorgesehen, der so breit wie der Halteabschnitt des Verbindungsstab 1 ist.

In den Figuren 4, 5 und 6 sind die proximalen Enden des Aufnahmekörpers 20, des Sperrelements 30 und des Klemmkörpers 40 vergrößert dargestellt. Der Aufnahmekörper 20 ist in Figur 6 gezeigt. Die beiden Führungsnuten 24 sind an den Seiten der Aufnahmeaussparung 22 angeordnet. Der erste Bereich mit den Nuten in der Axialrichtung ist am Eingangsbereich angeordnet und kann leicht durch Bohren oder Fräsen hergestellt werden. Die Führungsnut im zweiten Bereich ist wesentlich schwerer herzustellen, da sie vorzugsweise eine Krümmung aufweist. Eine effektive Methode diesen zweiten Bereich der Führungsnut bereitzustellen ist eine Durchgangsbohrung 23 wie es in den Figuren 2 und 6 dargestellt ist. Am proximalen Ende des Aufnahmekörpers ist eine verschließbare Montageöffnung 19. Die Montageöffnung 19 ist als Klappe ausgestaltet und ermöglicht das Einschieben und herausziehen der in dem Aufnahmekörper 20 befindlichen Teile (Klemmkörper 40 und Sperrelement 30). Diese Montageöffnung ist mit einer Klappe verschließbar, so dass die Einzelteile nicht aus dem Aufnahmekörper herausfallen können.

Der Aufnahmekörper 20 ist hohl ausgebildet, um das Sperrelement 30 und den Klemmkörper aufzunehmen. Auf seiner Innenseite weist der Aufnahmekörper 20 vorzugsweise einen Vorsprung auf, der als Positionierungsstift 27 dient. Dieser wirkt mit einer Positionierungsfläche 35 des Sperrelements 30 zusammen und legt das Sperrelement in Umfangsrichtung in dem Aufnahmekörper fest. Der Positionierungsstift 27 ist an seinem Ende abgeflacht und liegt lose an der Positionierungsfläche 35 an, so dass sich das Sperrelement 30 nicht in dem Aufnahmekörper 20 verdrehen kann.

Das Sperrelement 30 ist innerhalb des Aufnahmekörpers angeordnet. Er ist hohl und weist vorzugsweise einen U- oder O-förmigen Querschnitt auf. Am distalen Ende weist das Sperrelement 30 eine Sperre 32 auf, die hier als ein Vorsprung 32 ausgebildet ist, der zum Blockieren des in der Endstellung befindlichen Führungsbolzens 3 dient. Der Vorsprung ist hier zweigeteilt, damit das Sperrelement um den Verbindungsstab herumgreifen und über die Bolzen gelangen kann. Der Vorsprung 32 kann auf der oberen Seite an seinem Ende geneigt ausgebildet sein, damit die Führungsbolzen beim später beschriebenen Einrasten in die Festsitzposition das Sperrelement 30 wegdrücken oder zumindest das Wegziehen mittels eines Schiebehebels 36 unterstützen können. Auf seiner Innenseite weist das Sperrelement 30 vorzugsweise einen Vorsprung auf, der als Positionierungsstift 37 dient. In Figur 7 ist zu sehen, dass sich der Stift 37 in der gezeigten Ausführungsform am Schiebehebel 36 befindet. Dieser Positionierungsstift 37 wirkt mit einer Positionierungsfläche 45 des Klemmkörpers 40 zusammen und legt den Klemmkörper 40 in Umfangsrichtung in dem Sperrelement 30 fest. Der Positionierungsstift 37 ist an seinem Ende abgeflacht und liegt lose an der Positionierungsfläche 45 an, so dass sich der Klemmkörper 40 nicht in dem Sperrelement 30 verdrehen kann.

Am proximalen Ende ist das Sperrelement federnd gelagert. Die Feder 34 ist am Aufnahmekörper im Bereich des Griffs gelagert und spannt das Sperrelement 30 in die Sperrposition vor. Diese Feder 29 kann zwischen dem Aufnahmekörper 20 und dem Sperrelement 30 angeordnet sein, so dass sich das ganze Sperrelement in dem, Aufnahmekörper 20 bewegen kann. Sie kann aber auch als Teil des Sperrelements 30 ausgebildet sein. In diesem Fall ist das Sperrelement aus zueinander bewegbaren Einzelteilen ausgestaltet, so dass die Sperre 32 auf dem Sperrelement bewegbar gelagert ist und die Feder zwischen dem bewegbaren Teil mit der Sperre 32 und dem sich relativ zum Aufnahmeelement nicht bewegenden Teil angeordnet ist. Diese Lösung ist in Figur 3 dargestellt.

Um das Sperrelement 30 aus der Sperrposition (in der der Verbindungsstab 1 in der Festsitzposition gehalten wird) in eine offene Position (in der der Verbindungsstab 1 aus der Festsitzposition herausgenommen werden kann) zu bringen weist das Sperrelement 30 einen Schiebehebel 36 auf, mit dem das Sperrelement 30 innerhalb des Aufnahmeelements 20 verschoben werden kann. Der Schiebehebel 36 ist vorzugsweise am hinteren Ende angebracht und geht durch eine dort vorgesehene Aussparung am Aufnahmekörper hindurch. So kann der Anwender einhändig bequem den Schiebehebel 30 betätigen ohne dass der Griff an der Stabeinbringvorrichtung 10 gelockert oder gelöst werden müsste .

Der Klemmkörper 40 ist ebenfalls vorzugsweise innerhalb des Aufnahmekörpers 20 und des Sperrelements 30 angeordnet. Er ist insbesondere als länglicher und solider Stab ausgebildet, der an seinem proximalen Ende an der Kontaktstelle 26 gegen den Betätigungshebel 28 zur Verstärkung der Klemmkraft stößt und an seinem distalen Ende eine Klemmgeometrie aufweist. Diese ist dafür vorgesehen einen Kraftschluss mit dem proximalen Ende 2 des Verbindungsstabes 1 zu bilden. Grundsätzlich kann für eine beliebige Winkeleinstellung des Verbindungsstabs 1 der Kraftschluss lediglich durch Anpressen des Klemmkörpers 40 an das proximale Ende 2 des Verbindungsstabs 1 ausgeführt werden. Dazu kann der Klemmkörper eine sich verjüngende Geometrie aufweisen, beispielsweise einen Zahn oder eine Spitze. So wird die Klemmkraft auf einen kleinen Punkt reduziert und erhöht. Wenn für die Winkelstellung allerdings auch eine stufenweise Einstellung (also in vorgegebenen Winkelschritten) ausreicht, dann kann die Sicherheit des Kraftschlusses und vor allem die Sicherheit gegen ein unabsichtliches Verdrehen der Winkelstellung erhöht werden. Dies kann beispielsweise geschehen, wenn das proximale Ende 2 des Verbindungsstabs1 an seinem Umfang flache Bereiche aufweist, so dass der Klemmkörper am distalen Ende ebenfalls flach ausgebildet sein kann. Bevorzugt ist jedoch, dass das Ende mit einer Zahngeometrie versehen ist, die insbesondere an einer konkaven, nach innen gewölbten Fläche am distalen Ende vorgesehen ist. Die Zahngeometrie ist in Figur 4 inklusive der konkaven Wölbung des Endes des Klemmkörpers dargestellt. Das proximale Ende 2 des Verbindungsstabs 1 weist dann eine komplementäre Oberfläche mit Einkerbungen auf, in die die Zähne eingreifen können.

Bei der Anwendung der in den Figuren gezeigten Stabeinbringvorrichtung 10 wird die Vorrichtung zuerst montiert. Dazu wird der Betätigungshebel 28 in eine Montagestellung gebracht, so dass er für die Montage nicht im Weg ist. Für die Montagestellung wird die Feder 29 aus dem Betätigungshebel 28 ausgehängt und der Hebel 28 ganz nach oben weg vom Handgriff geklappt. Dann wird das Sperrelement 30 durch die Montageöffnung 19 in den Aufnahmekörper 20 eingeschoben und dann der Klemmkörper 40 in das Sperrelement 30. Je nach Ausgestaltung des Sperrelements 30 kann dann die Feder über den Klemmkörper 40 geschoben werden. Dann wird die Montageöffnung 19 mit der Klappe 18 verschlossen und der Betätigungshebel 28 in die Betätigungsposition heruntergeklappt und in die Feder 29 eingerastet. Als nächstes wird der Verbindungsstab 1 in die Aufnahmeaussparung 22 in den ersten Bereich der Führungsnut 24 eingeführt. Dann schlagen die Bolzen 3 gegen den Sperrvorsprung 32, der entweder über die geneigte Ausgestaltung weggeschoben wird oder der mittels des Schiebehebels 36 nach hinten gezogen wird. Die Führungsbolzen 3 werden automatisch in die zweite Richtung weiter in die Festsitzposition in der Führungsnut 24 geschoben und das Sperrelement 30 bzw. die Sperre 32 rastet wieder in die Sperrstellung ein. Der Verbindungsstab 1 ist nun in der Festsitzposition festgelegt aber immer noch um die Achse der Führungsbolzen 3 verdrehbar. Dann wird die gewünschte Winkelstellung des Verbindungsstabs 1 eingestellt der Betätigungshebel 28 mit den Fingern angezogen, so dass der Klemmkörper 40 gegen das proximale Ende 2 des Verbindungsstabs 1 gedrückt und in der Winkelstellung fixiert wird. Dabei wird der Kraftschluss des Endes des Klemmkörpers mit dem proximale Ende 2 des Verbindungsstabs 1 erreicht um ein Verdrehen des Verbindungsstabs zu verhindern. Der Betätigungshebel 28 kann dann in seiner angezogenen Stellung fixiert werden. Nach diesen Schritten kann der Verbindungsstab eingebracht werden. Wiederholtes Lösen/Fixieren des Verbindungsstabs 1 erlaubt eine schrittweise Einbringung des Verbindungsstabs 1 in die Pedikelschraube(n). Je nach Ausgestaltung des Klemmende 42 und komplementär des proximalen Endes 2 des Verbindungsstabs 1 ist dies stufenlos oder in vorgegebenen Winkelpositionen möglich.

Zum Lösen des Verbindungsstabs 1 wird der Betätigungshebel 28 wieder gelöst und durch die Feder 29 nach vorne gedrückt, dadurch wird der Kraftschluss gelockert, so dass eine leichte Bewegung ausreicht um den Eingriff des Klemmkörpers 40 an dem proximalen Ende 2 des Verbindungsstabs 1 zu lösen. Zum Diskonnektieren der Stabeinbringvorrichtung wird der vor der Feder 34 befindliche Teil des Sperrelements 30am Schiebehebel 36 zurückgezogen. Dadurch geben die Sperrvorsprünge 32 die Bolzen 3 des Verbindungsstabs 1 frei, diese gleiten entlang der Führungsnut aus der Festsitzposition in den ersten Bereich der Führungsnut und die Stabeinbringvorrichtung 10 kann vom Verbindungsstab 1 abgezogen werden. Zum Reinigen wird dann die Montageöffnung geöffnet und die Einzelteile werden aus dem Aufnahmeelement herausgenommen.

**Bezugszeichenliste**

| | |
|---|---|
| Verbindungsstab | 1 |
| Proximales Ende | 2 |
| Bolzen | 3 |
| konische Spitze | 5 |
| gebogener Abschnitt | 7 |
| Halteabschnitt | 9 |
| | |
| Stabeinbringvorrichtung | 10 |
| Handgriff | 12 |
| Klappe | 18 |
| Montageöffnung | 19 |
| | |
| Aufnahmekörper | 20 |
| Schlitz | 21 |
| Aufnahmeaussparung | 22 |
| Durchgangsbohrung | 23 |
| Führungsnuten | 24 |
| Kontaktstelle | 26 |
| Positionierungsstift | 27 |
| Betätigungshebel | 28 |
| Feder | 29 |
| | |
| Sperrelement | 30 |
| Sperrvorsprung | 32 |
| Feder | 34 |
| Positionierungsfläche | 35 |
| Schiebehebel | 36 |
| Positionierungsstift | 37 |
| | |
| Klemmkörper | 40 |
| Klemmende | 42 |
| Positionierungsfläche | 45 |

## Patentansprüche

1. Stabeinbringvorrichtung (10) zum Einbringen eines Verbindungsstabs (1) in eine Pedikelschraube,
umfassend einen Aufnahmekörper (20), der an seinem distalen Ende eine Aufnahmeaussparung (22) mit gegenüberliegenden Führungsnuten (24) aufweist;
einen in dem Aufnahmekörper (20) angeordneten Klemmkörper (40) zum Festklemmen des Verbindungsstabs (1) in der Aufnahmeaussparung (22);
**dadurch gekennzeichnet, dass**
die Führungsnuten (24) der Aufnahmeaussparung (22) in einem Eingangsbereich in einer ersten Richtung in dem Aufnahmekörper (20) ausgebildet sind und in einem Festsitzbereich nach dem Eingangsbereich in einer zweiten Richtung unterschiedlich zur ersten Richtung ausgebildet sind, so dass ein im Festsitzbereich positionierter Verbindungsstab (1) in der ersten Richtung blockiert ist und/oder dass ferner ein Sperrelement (30) mit einer Sperre (32) vorgesehen ist, die zwischen einer Freigabeposition und einer Sperrposition bewegbar gelagert ist.

2. Stabeinbringvorrichtung (10) nach Anspruch 1, bei der das Sperrelement entlang einer Eingangsrichtung der Aufnahmenut verschiebbar gelagert ist.

3. Stabeinbringvorrichtung (10) nach Anspruch 2, bei der an dem distalen Ende des Sperrelements zumindest ein Sperrvorsprung vorgesehen ist, der in der Sperrposition über die Führungsbolzen (3) ragt.

4. Stabeinbringvorrichtung (10) nach Anspruch 2 oder 3, bei der das Sperrelement in dem Aufnahmekörper (20) federnd gelagert und in die Sperrposition vorgespannt ist.

5. Stabeinbringvorrichtung (10) nach Anspruch 2, 3 oder 4, bei der das Sperrelement am proximalen Ende einen Schiebehebel zum Verschieben aufweist.

6. Stabeinbringvorrichtung (10) nach einem der vorhergehenden Ansprüche, bei dem der Klemmkörper (40) an seinem distalen Ende (42) eine sich verjüngende Geometrie oder eine Zahngeometrie aufweist.

7. Stabeinbringvorrichtung (10) nach einem der vorhergehenden Ansprüche, bei der der Aufnahmekörper (20), der Klemmkörper (40) und insbesondere auch das Sperrelement (30) als sich längserstreckende Zylinderkörper ausgebildet sind.

8. Stabeinbringvorrichtung (10) nach Anspruch 7, bei dem der Klemmkörper (40) im Sperrelement und das Sperrelement in dem Aufnahmekörper (20) angeordnet sind.

9. Stabeinbringvorrichtung (10) nach einem der vorhergehenden Ansprüche, bei der am Aufnahmekörper (20) der Haltegriff als Pistolengriff ausgebildet ist.

10. Stabeinbringvorrichtung (10) nach einem der vorhergehenden Ansprüche, bei der das Ende des Aufnahmekörpers (20) am proximalen Ende eine verschließbare Montageöffnung (19) aufweist.

11. Stabeinbringvorrichtung (10) nach einem der vorhergehenden Ansprüche, bei der der Klemmkörper (40) mittels eines Betätigungshebel (28) gegen einen Klemmbereich des Verbindungsstabs (1) gepresst wird.

## Claims

1. Rod introducing device (10) for introducing a connecting rod (1) into a pedicle screw,
comprising a receiving body (20), which has a receiving recess (22) with opposing guide grooves (24) at its distal end;
a clamping body (40) arranged in the receiving body (20) for clamping the connecting rod (1) in the receiving recess (22);
**characterized in that**,
the guide grooves (24) of the receiving recess (22) are formed in the receiving body (20) in a first direction in an inlet region and in a second direction which differs from the first direction in a securing region after the inlet region such that a connecting rod (1) positioned in the securing region is blocked in the first direction and/or a blocking element (30) with a blocking means (32) is additionally provided, said blocking means being mounted in a movable manner between a release position and a blocking position.

2. Rod introducing device (10) according to claim 1, wherein the blocking element is mounted displaceable along an entry direction of the receiving groove.

3. Rod introducing device (10) according to claim 2, wherein, at the distal end of the blocking element, at least one blocking protrusion is provided, which protrudes above the guide bolts (3) in the blocking position.

4. Rod introducing device (10) according to claim 2 or 3, wherein the blocking element is mounted spring-loaded in the receiving body (20) and is pre-stressed in the blocking position.

5. Rod introducing device (10) according to claim 2, 3 or 4, wherein, at the proximal end, the blocking element comprises a slide lever for displacement.

6. Rod introducing device (10) according to any one of the preceding claims, wherein the clamping body (40) has at its distal end (42) a tapering geometry or a tooth geometry.

7. Rod introducing device (10) according to any one of the preceding claims, wherein the receiving body (20), the clamping body (40) and in particular also the blocking element (30) are formed as longitudinally extending cylinder bodies.

8. Rod introducing device (10) according to claim 7, wherein the clamping body (40) is arranged in the blocking element and the blocking element is arranged in the receiving body (20).

9. Rod introducing device (10) according to any one of the preceding claims, wherein the handle is formed as a pistol grip on the receiving body (20).

10. Rod introducing device (10) according to any one of the preceding claims, wherein the end of the receiving body (20) comprises at the proximal end a closable assembly opening (19).

11. Rod introducing device (10) according to any one of the preceding claims, wherein the clamping body (40) is pressed against a clamping area of the connecting rod (1) by means of an operating lever (28).

## Revendications

1. Dispositif d'insertion de tige (10) pour insérer une bielle (1) dans une vis pédiculaire, comprenant un corps de réception (20), ayant à son extrémité distale un évidement de réception (22) avec des rainures de guidage opposées (24);
un corps de serrage (40) disposé dans le corps de réception (20) pour le serrage de la bielle (1) dans l'évidement de réception (22);
**caractérisé en ce que**
les rainures de guidage (24) de l'évidement de réception (22) dans une zone d'entrée sont formées dans une première direction dans le corps de réception (20) dans une zone d'entrée et sont formées différemment de la première direction dans une zone de siège fixe après la zone d'entrée dans une seconde direction, de sorte qu'une bielle (1) placée dans la zone de siège fixe est bloquée dans la première direction et/ou **en ce que**, en outre, un élément de blocage (30) est prévu avec un bloc (32), qui est monté mobile entre une position de libération et une position de blocage.

2. Dispositif d'insertion de tige (10) selon la revendication 1, dans lequel l'élément de blocage est monté de manière mobile le long d'une direction d'entrée de la rainure de réception.

3. Dispositif d'insertion de tige (10) selon la revendication 2, dans lequel au moins une saillie de verrouillage est prévue à l'extrémité distale de l'élément de verrouillage, laquelle saillie de verrouillage dépasse des goupilles de guidage (3) dans la position de blocage.

4. Dispositif d'insertion de tige (10) selon la revendication 2 ou 3, dans lequel l'élément de blocage est monté élastiquement dans le corps de réception (20) et précontraint dans la position de blocage.

5. Dispositif d'insertion de tige (10) selon la revendication 2, 3 ou 4, dans lequel l'élément de blocage présente un levier coulissant de déplacement à l'extrémité proximale.

6. Dispositif d'insertion de tige (10) selon l'une des revendications précédentes, dans lequel le corps de serrage (40) présente une géométrie conique ou une géométrie de dent à son extrémité distale (42).

7. Dispositif d'insertion de tige (10) selon l'une des revendications précédentes, dans lequel le corps de réception (20), le corps de serrage (40) et en particulier l'élément de blocage (30) sont conçus comme des corps cylindriques s'étendant longitudinalement.

8. Dispositif d'insertion de tige (10) selon la revendication 7, dans lequel le corps de serrage (40) est disposé dans l'élément de blocage et l'élément de blocage est disposé dans le corps de réception (20).

9. Dispositif d'insertion de tige (10) selon l'une des revendications précédentes, dans lequel la poignée du corps récepteur (20) est conçue comme une poignée pistolet.

10. Dispositif d'insertion de tige (10) selon l'une des revendications précédentes, dans lequel l'extrémité du corps récepteur (20) présente une ouverture de montage (19) pouvant être fermée à l'extrémité proximale.

11. Dispositif d'insertion de tige (10) selon l'une des revendications précédentes, dans lequel le corps de serrage (40) est pressé au moyen d'un levier de commande (28) contre une zone de serrage de la bielle (1).
